# EUROPÄISCHE PATENTANMELDUNG

(11) **EP 1 126 271 A1**
(43) Veröffentlichungstag der Anmeldung: **22.08.2001**
(21) Anmeldenummer: 00102957.8
(22) Anmeldetag: 14.02.2000
(51) Int. Cl.: G01N 21/64, G01N 33/48, A61B 5/00

(54) **Verfahren und Vorrichtung zur Bestimmung der Malignität von Tumorgewebe und zur Auswahl von Substanzen, die das Gewebe günstig beeinflussen**

(71) Anmelder: Popp, Fritz-Albert, Dr.rer.nat.habil., Visiting Professor (mult.), 41472 Neuss (DE)
(72) Erfinder: Popp, Fritz-Albert, Dr.rer.nat.habil., Visiting Professor (mult.), 41472 Neuss (DE)
(74) Vertreter: Frommhold, Joachim, Dr.

(57) **Zusammenfassung**

Die Erfindung betrifft ein Verfahren sowie eine Einrichtung zur Bestimmung der Malignität und Reaktivität von Tumorgewebe, insbesondere in der Humanmedizin. An Hand gemessener Lichtemissionswerte des Gewebes ist es möglich, geeignete Agenzien zur Behandlung des Gewebes auszuwählen und anzuwenden.

## Beschreibung

Die Erfindung betrifft ein Verfahren zur Bestimmung der Malignität von Tumorgewebe, bei dem
a1) die "ultraschwache Lichtemission" (Biophotonen) des Tumorgewebes mit einem hinreichend empfindlichen Lichtdetektor registriert wird und mindestens ein charakteristischer Parameter dieser Lichtabstrahlung gemessen wird; und/oder
a2) das Tumorgewebe mit Licht bestrahlt wird, die Bestrahlung beendet wird und wenigstens eine charakteristische Größe der Lichts, das von dem Gewebe nach Beendigung der Bestrahlung emittiert wird, gemessen wird; und/oder
a3) das Tumorgewebe mit anderen physikalischen Methoden (zum Beispiel Ultraschall) oder Agenzien behandelt wird und nach der Behandlung das Licht nach einer der Methoden al) und a2) gemessen wird, und
a4) das Tumorgewebe insbesondere mit nicht-toxischen Agenzien und/oder Arzneimitteln behandelt wird und während oder nach der Behandlung das Licht nach einer der Methoden al), a2) und a3) gemessen wird, und
b1) aus der (oder den) gemessenen charakterischen Größe(n) des Lichts auf die Malignität des Tumorgewebes geschlossen wird, und nach Anwendung des Verfahrens a4) insbesondere auch
b2) aus der (oder den) gemessenen charakteristischen Größe(n) des Lichts auf die günstigste Wirksamkeit, auf eventuelle Unwirksamkeit oder auf mögliches "enhancement" getesteter nicht-toxischer Arzneimittel geschlossen wird.

Die Meßverfahren sind aus der EP 0 430 150 A1 bekannt.

Bekannt gemacht wurde der erste experimentelle Nachweis der spontanen Lichtemission aus biologischen Systemen ("Biophotonen") zum Beispiel in der Veröffentlichung B. Ruth und F.A.Popp: Experimentelle Untersuchungen zur ultraschwachen Photonenemission biologischer Systeme. Z.Naturforsch.31c (1976), 741-745. Eine entsprechende Anlage zur Messung ist beispielsweise aus der DE 94 17 845 U1 bekannt.
Die Erfindung ist auch auf die Verwendung einer Lichtemissions-Meßeinrichtung zur Durchführung des - unten näher erläuterten - erfindungsgemäßen Verfahrens gerichtet.

Gegen Mitte der 70-er Jahre wurde erstmalig (siehe die o.g. Veröffentlichung von B. Ruth und F.A.Popp) experimentell nachgewiesen, daß pflanzliche und tierische Zellen fortwährend spontan Photonen im Wellenlängenbereich von 200 bis 800 nm emittieren. Die Intensität liegt in der Größenordnung von wenigen bis einigen hundert Photonen/(s x cm²), entsprechend einer Strahlungsleistung im Größenordnungsbereich von 10⁻¹⁷ W. Es handelt sich dabei nicht etwa um zufällig emittierte Lichtquanten stochastischen Ursprungs. Vielmehr folgt die Photonenzählstatistik einer Poisson-Verteilung und die Lichtemission nach äußerer Anregungdie sogenannte verzögerte Lumineszenz - einer hyperbolischen (und für gesunde Zellen nicht etwa einer exponentiellen!) Abklingcharakteristik. Beide Merkmale zusammengenommen belegen, daß es sich um Licht aus einem kohärenten elektromagnetischen Feld handelt (z.B. F.A.Popp and K.H.Li: Hyperbolic relaxation as a sufficient condition of a fully coherent ergodic field. International Journal of Theoretical Physics. Vol. 32, No.9 (1993), 1573-1583).

Änderungen im biologischen oder physiologischen Zustand eines lebenden Systems spiegeln sich in einer entsprechenden Änderung der Biophotonen-Emission oder der verzögerten Lumineszenz wider. Auch wurden deutliche Hinweise dafür gefunden, daß die Kommunikation und Organisation von Zellen zumindest teilweise durch Biophotonen vermittelt wird.

Der Erfinder hat erkannt, daß die Verwendung von Meßgeräten, die sowohl diese "ultraschwache Photonenemission" registrieren können als auch die "verzögerte Lumineszenz" die Möglichkeit bietet, die Qualität selbst, besonders aber auch schon geringfügige Änderungen der Qualität untersuchter biologischer Systeme mit höchster Präzision und Empfindlichkeit, oft in nichtinvasiver Weise schnell und sicher zu erfassen und wertvolle Schlüsse daraus zu ziehen. Beispiele und Charakteristika der ultraschwächen Photonenemission und der verzögerten Lumineszenz, die sich zur Auswertung eignen, sind in EP 0 430 150 A1 benannt. Insbesondere läßt sich die Methode auch dafür nutzen, um Tumorgewebe von gesundem Gewebe eindeutig zu unterscheiden (F.A.Popp: DE 2844217: Verfahren zur Diagnose der Malignität von menschlichem, tierischen und pflanzlichen Gewebe). Dieses Verfahren funktioniert auch in beschränktem Umfang bei wesentlich höheren Strahlungsleistungen der Lichtanregung, so daß die Detektorsysteme nicht für alle Fälle die hohe Empfindlichkeit aufweisen müssen wie nach der Methode der Erfinder Popp und Ruth. In dieser modifizierten Form hoher Lichtleistungen wird die Methode in der Praxis auch bereits eingesetzt ( Hinweise zum Beispiel in dem beigefügten Artikel von Kathrin Lenzer: Krebszellen strahlen wie Glühwürmchen, aus der Neuß-Grevenbroicher Zeitung vom 21.10.1999). Diese abgewandelte Methode beruht ebenfalls auf den Erkenntnissen, die zum Patent DE 2844217 geführt haben.

In der ursprünglichen Form, nämlich Biophotonen und verzögerte Lumineszenz mit solchen Meßgeräten zu messen, die noch im Intenstitätsbereich von 10⁻¹⁷ Watt empfindlich sind, bietet die Methode aber nach wie vor erhebliche Vorteile gegenüber abgewandelten Verfahren bei höheren Strahlungsleistungen, die keine besonders hohe Empfindlichkeit der Lichtmeßgeräte voraussetzen. Der Erfinder hat erkannt, daß diese Vorteile darin liegen, praktisch *alle* Tumorgewebe - und nicht nur jene mit besonders auffälligen Reemissions-Intensitäten - erfassen zu können, ferner den Malignitätsgrad des Gewebes feststellen zu können und darüberhinaus Agenzien austesten zu können, die für die Heilung unter Umständen entscheidende Hilfe erwarten lassen. Die Messungen, die in den letzten Jahren an Tumorgeweben nach dem beschriebenen Verfahren (Messung der spontanen Biophotonenemission und der verzögerten Lumineszenz mit Detektoren einer Empfindlichkeit um 10⁻¹⁷ W) durchgeführt wurden, bestätigten nicht nur die Erkenntnisse, die im Patent DE 28 44 217 ihren Niederschlag fanden, sondern zeigten überraschend, daß es gelingt,
■ individuell den Malignitätsgrad des Tumorgewebes zu testen,
■ individuell nicht-toxische Agenzien auf ihre Wirksamkeit in der Reduktion der Malignität des Tumorgewebes zu prüfen.

Der Erfindergedanke beruht darauf, daß Behandlungen, die die Lichtemission des Tumorgewebes in Richtung der Lichtemission des Normalgewebes verändern, hilfreich sind, während jene, die zu einem stärkeren Unterschied zwischen Normalgewebe und Tumorgewebe führen, unter gewissen Umständen vermieden werden sollten.

Es zeigte sich auch tatsächlich, daß selbst bei gleicher Tumor*art* der Tumor *verschiedener* Patienten praktisch in allen Stadien des Fortschreitens der bösartigen Erkrankung *verschiedene, aber für die jeweilige Person konstante und spezifische* Biophotonenabstrahlungen und charakteristische Lichtintensitäten der "verzögerten Lumineszenz" aufweist. Merkmale der Photonenemission, zum Beispiel die Intensität des Nachleuchtens in bestimmten Wellenlängenbereichen, lassen wertvolle Schlüsse auf die Malignität der Tumoren und ihre Metastasierungsfreudigkeit zu. Wie aus dem Patent DE 28 44 217 bekannt ist, ist die Malignität im allgemeinen umso höher zu bewerten, je höher die Photonenzählraten des Tumorgewebes sind. Es zeigt sich darüber hinaus, daß die Licht-Abstrahlung der Tumoren verschiedener Patienten *selbst bei gleicher Tumorart individuell verschieden auf nicht-toxische Agenzien*, *und zwar relativ unabhängig vom Entwicklungsstadium des Tumors*, reagiert. In den meisten Fällen steigt die Strahlungsintensität des Tumorgewebes nach Zugabe nicht-toxischer Agenzien allerdings an. Das gilt auch für die meisten nicht-toxischen Arzneimittel, die heute in der Tumortherapie verabreicht werden. Es erweist sich aber auch umgekehrt, daß man im individuellen Fall, nicht jedoch allgemein für jede Tumorart, durehaus nicht-toxische Arzneimittel finden kann, die anstatt eines Anstiegs eine deutliche Reduktion der Lichtintensität und anderer Charakteristika der Photonenemission in Richtung des gesunden Gewebes bewirken. Diese Reaktion ist - wie mehrfach betont - vom individuellen Tumor des Patienten abhängig. Sie ändert sich nur wenig mit der Tumorprogression. Sie hängt nicht nur vom Wirkstoff selbst, sondern auch von der Konzentration des verwendeten Wirkstoffs ab. Sie fällt selbst bei gleichen Tumorarten für verschiedene Patienten für gleiche Agenzien unter Umständen stark verschieden aus. Es ist anzunehmen, daß diese wenigen, individuell anzupassenden nicht-toxischen Agenzien, die die Photonenemission des Tumorgewebes eines Patienten in Richtung der Photonenemission des gesunden Gewebes verändern, eine positive Wirkung in der Behandlung des Tumors erzielen. Erfahrungen, die bisher gesammelt werden konnten, bestärken diese Vermutung.

Die Methode bietet demnach folgende Vorteile:
■ Sie erlaubt die Beurteilung des Malignitätsgrads (und der Metastasierungsfreudigkeit) des Tumorbefalls im Individualfall.
■ Sie ermöglicht die Zurückweisung unwirksamer oder schädlicher nicht-toxischer Arzneimittel im Individualfall.
■ Sie ermöglicht die Auswahl möglicherweise wirksamer nicht-toxischer Heilmittel im Individualfall.

Ein Anwendungbeispiel, das der praktischen Erfahrung entstammt, verdeutlicht die Nutzanwendung der beschriebenen Erfindung:

Der maligne und im allgemeinen metastasierende Tumor der Patientin R. wurde operiert. Fünf Teile des Tumorgewebes wurden in physiologiseher Kochsalzlösung in jeweils einer Quarzglasküvette (10 ml) bei Zimmertemperatur auf die Biophotonenemission und die verzögerte Lumineszenz untersucht. Der Reihe nach hatten die Proben 1-5 die folgenden Meßwerte:

| | | |
|---|---|---|
| 1 | PE 40 cps | DL 420 cps |
| 2 | PE 36 cps | DL 480 cps |
| 3 | PE 16 cps | DL 730 cps |
| 4 | PE 67 cps | DL 530 cps |
| 5 | PE 26 cps | DL 620 cps |

PE bedeuten die Mittelwerte über jeweils 256 Meßwerte der Spontanemission (in counts per second, cps), DL jene der Leuchtintensität nach vorheriger Anregung mit Weißlicht.

In die Küvetten 1 - 5 wurden dann jeweils gleiche Mengen von fünf verschiedenen flüssigen nicht-toxischen käuflich erwerbbaren Anti-Tumor-Arzneimitteln ( teilweise auf Mistelbasis) gegeben, und danach erneut die Lichtintensität gemessen. Es ergaben sich mit den fünf Medikamenten 1 - 5 die folgenden neuen Meßwerte:

| | | |
|---|---|---|
| 1 | PE 75 cps | DL 920 cps |
| 2 | PE 32 cps | DL 410 cps |
| 3 | PE 47 cps | DL 420 cps |
| 4 | PE 16 cps | DL 115 cps |
| 5 | PE 90 cps | DL 780 cps |

Das Ergebnis zeigt, daß zwei der Medikamente (nämlich 2 und 3) nahezu unwirksam sind, zwei weitere, nämlich 1 und 5, zu einer deutlichen Erhöhung der Lichtintensität führen, während das Medikament 4 eine signifikante Senkung der Lichtintensität in Richtung einer Normalisierung bewirkt. Die Medikamente 2 und 3 wären nach diesem Test als unwirksam einzustufen, die Medikamente 1 und 5 bergen das bekannte Risiko der Beschleunigung des Tumorwachstums ("enhancement"), während das Medikament 4 das Gewebe in Richtung normalen Gewebes zurückführen könnte.

Die Patientin bekam vom Arzt das Medikament 4 verordnet. Von weiteren Therapiemaßnahmen wurde abgesehen. Der Vorgang liegt heute zirka 12 Jahre zurück. Die klinischen Werte der Patientin, die heute als geheilt betrachtet wird, verbesserten sich sprunghaft nach etwa einem Jahr konsequenter Einnahme des Medikaments.

Damit ist sicher noch kein Beweis für die Wirksamkeit erbracht. Die bisherigen weiteren Erfahrungen deuten aber darauf hin, daß diese Methode ein neues und brauchbares Verfahren für die nicht-invasive Analyse maligner Erkrankungen darstellt.

## Patentansprüche

1. Verfahren zur Bestimmung der Malignität und Reaktivität von Tumorgewebe auf externe Behandlung, bei dem
a1) die ultraschwache Lichtemission (Biophotonen) des Gewebes gemessen wird, und/oder
a2) das Gewebe mit Licht bestrahlt wird und wenigstens eine charakteristische Größe der Licht-Reemission nach Beendigung der Lichtbestrahlung (verzögerte Lumineszenz) gemessen wird, und/oder
a3) das Gewebe mit anderen Methoden (zum Beispiel Ultraschall, Wärme) angeregt wird und charakteristische Größen der Lichtemission nach al) und oder a2) gemessen werden;
a4) das Gewebe mit verschiedenen Methoden (z.B. Zugabe nicht-toxischer Agenzien) behandelt wird, und /oder die Lichtemission mit mindestens einer der Methoden a1), a2) oder a3) gemessen wird, und/oder
a5) das Gewebe mit mindestens einer der Methoden al), a2) oder a3) bei verschiedenen Zelldichten gemessen wird, und
b1) aus der Lichtemission auf die Malignität des Gewebes, seine Reaktivität und Metastasierungsfreudigkeit geschlossen wird, und
b2) aus den Ergebnissen der Lichtemission nach a4) und eventuell auch nach a5) Hinweise auf die Wirksamkeit getesteter Behandlungsmethode verwertet werden.

2. Verfahren nach Anspruch 1, bei dem nichtbefallenes Gewebe als Kontrolle mitverwendet wird.

3. Verfahren nach Anspruch 1 und 2, bei welchem die zur Meßwerterlangung dienenden Schritte a1) und /oder a2) und ggf. auch die Schritte b1) und b2) mehrfach nacheinander ausgeführt werden und die hierdurch gewonnenen Ergebnisse zur Erhöhung der Genauigkeit der ermittelten Malignität und Reaktivität des Gewebes zusammengefaßt werden.

4. Verfahren nach einem der Anspräche 1 bis 3, bei dem eine oder mehrere der folgenden charakteristischen Lichtgrößen verwendet werden:
■ der Intensitätswert der spontanen Emission (Biophotonenemission) des Gewebes,
■ der Intensitätswert des im ersten Meßzeitintervall nach Anregung emittierten Lichts,
■ die Abklingfunktion der Lichtemission nach Lichtanregung,
■ spektrale Charakteristika der Lichtemission (zum Beispiel Intensität in bestimmten Wellenlängenintervallen),
■ die Photonenzählstatistik (Häufigkeit, bestimmte Photonenzahlen in vorgegebenen Meßzeitintervallen anzutreffen).

5. Anlage zur Bestimmung der Malignität und Reaktivität von Tumorgewebe mit einer Einrichtung, die sowohl die Messung der spontanen Emission (Biophotonen) als auch die der. verzögerten Lumineszenz zuläßt, wobei die Empfindlichkeit ausreicht, um eine Intensität von mindestens etwa tausend Quanten pro Sekunde zu registrieren.

6. Anlage nach Anspruch 5 zur Bestimmung der Malignität und Reaktivität von Tumorgewebe, gekennzeichnet durch eine Auswerteeinrichtung, welche aus den Charakteristika der gemessenen Lichtemissionswerte die Malignität und Reaktivität des Gewebes bestimmt.

7. Anlage nach Anspruch 5 und 6, welche durch eine Vorrichtung gekennzeichnet ist, die die Messung der Lichtcharakteristika von Gewebe während der Behandlung mit Agenzien erlaubt.
